# EUROPEAN PATENT APPLICATION

(11) **EP 4 501 385 A1**
(43) Date of publication of application: **05.02.2025**
(21) Application number: 24191040.5
(22) Date of filing: 26.07.2024
(51) Int. Cl.: A61M 25/01, A61B 17/00

(54) **CATHETER WITH TACTILE INDICATION OF DEFLECTION**

(30) Priority: 28.07.2023 US 202318227565
(71) Applicant: Biosense Webster (Israel) Ltd., 2066717 Yokneam (IL)
(72) Inventor: PHAM, Cuong, Irvine, CA 92618 (US); SOLTE, Isabel, Irvine, CA 92618 (US); JOSHI, Helee, Irvine, CA 92618 (US); NGUYEN, Thanh, Irvine, CA 92618 (US); DATTA, Keshava, Irvine, CA 92618 (US)
(74) Representative: Carpmaels & Ransford LLP

(57) **Abstract**

A catheter (10) with tactile indication of deflection, comprising a shaft (129, a control handle (16) with a housing configured with an indent (44), and a deflection lever (22) mounted on the housing over the indent (44) and configured to deflect the shaft with rotation of the deflection lever. The deflection lever (22) includes a ball bearing (42) and a spring screw (40) configured to mobilize the ball bearing into elastic contact with the indent when the deflection lever is rotated into a neutral position, where the engagement between the ball bearing (42) and the indent (44) provides a tactile indication of shaft deflection detected by an operator holding the deflection lever.

## Description

### FIELD OF INVENTION

This invention relates to catheters with deflectable shafts, in particular, catheters with control handles equipped with deflection levers.

### BACKGROUND

Cardiac arrhythmia is irregular beating of the heart caused by aberrant electrical signals. Arrhythmias can reduce quality of life and carry increased risk of stroke and heart failure. Arrythmias can be located and identified via diagnostic catheters. These catheters can be used to create electroanatomical maps to help electrophysiologists understand the pathology and plan and deliver therapy which can include ablation via therapeutic catheters.

A conventional electrophysiology (EP) catheter, whether diagnostic or therapeutic, is steered through a patient's vascular by an operator manipulating a control handle. The catheter typically has one or more puller wires that extend through a catheter shaft between a control handle and a distal effector end, with mechanisms in the control handle that act on proximal ends of the puller wires to effectuate deflection of the catheter shaft. The catheter may include a thumb control that slides longitudinally relative to the control handle typically for unidirectional deflection of the catheter shaft, or the catheter may include a deflection lever that rotates from side to side for bidirectional deflection of the catheter shaft. In the latter regard, the deflection lever has two opposing arms, one on each side of the control handle, and is configured to occupy a "neutral" position with no deflection of the catheter shaft when the arms extend evenly on each side of the control handle. With a thumb and an index finger on a respective arm of the deflection lever, an operator can rotate the deflection lever with either the thumb or index finger to deflect the catheter shaft in that same direction. While the deflection lever may have a visual marker thereon indicating the direction of rotation, the operator is primarily viewing a 3D graphical anatomical map displaying the position of the catheter distal end. Moreover, manipulation of the catheter distal end may involve the operator flipping the control handle upside down such that the deflection lever is on the underside of the control handle, out of sight, and the direction of deflection is not apparent from the mere feel of the control handle. Thus, despite the presence of the visual marker on the deflection lever, the orientation of the deflection lever as an indicator of catheter deflection is not readily apparent to the operator in the midst of a procedure.

Accordingly, applicants recognized that there is a need to provide a deflection catheter that provides tactile indication of the orientation of the deflection lever and the state of deflection of the catheter shaft.

### SUMMARY OF THE DISCLOSURE

In some embodiments, a catheter with tactile indication of deflection, comprises a shaft, a control handle with a housing configured with an indent, and a deflection lever mounted on the housing over the indent and configured to deflect the shaft with rotation of the deflection lever. The deflection lever includes a ball bearing and a spring screw configured to mobilize the ball bearing into elastic contact with the indent when the deflection lever is rotated into a neutral position such that the elastic contact provides a tactile indication of shaft deflection detected through the deflection lever.

In some embodiments, the ball bearing has elastic contact with the housing when the deflection lever is rotated out of the neutral position.

In some embodiments, the indent is aligned with a longitudinal axis of the control handle.

In some embodiments, the deflection lever includes a boss through which the spring screw extends.

In some embodiments, the spring screw includes a head and an end that together define an axis that is generally perpendicular to the longitudinal axis, and the ball bearing is between the end and the indent.

In some embodiments, a catheter comprises a control handle including an outer surface with an indent and a deflection lever mounted on the control handle over the indent, the deflection lever being configured for rotation relative to the control handle. The catheter also includes a ball bearing, a spring screw configured to move the ball bearing in and out of engagement with the indent with rotation of the deflection lever, and a shaft distal of the control handle, the shaft responsive to the deflection lever for bidirectional deflection.

In some embodiments, the ball bearing is between the spring screw and the outer surface and the spring screw is configured to urge the ball bearing toward the outer surface.

In some embodiments, the control handle defines a longitudinal axis and the deflection lever is configured for rotation about an axis perpendicular to the longitudinal axis.

In some embodiments, the deflection lever is configured with a boss through which the spring screw extends.

In some embodiments, the deflection lever has a first arm with a tactilely smooth surface and a second arm with a tactilely ridged surface.

In some embodiments, the deflection lever is in a neutral position when the ball bearing is engaged with the indent.

In some embodiments, the deflection lever is in a rotated position when the ball bearing is out engagement with the indent.

In some embodiments, the shaft is undeflected when the deflection lever is in a neutral position.

In some embodiments, the shaft is deflected when the deflection lever is in a rotated position.

In some embodiments, a catheter comprises a control handle that defines a longitudinal bisecting axis and includes an outer surface with an indent aligned with the longitudinal bisecting axis. The catheter also includes a deflection lever that is mounted on the control handle and configured for bidirectional rotation relative to the longitudinal bisecting axis about a rotational axis perpendicular to the longitudinal bisecting axis, where the indent defines a neutral position of the bidirectional rotation of the deflection lever. The catheter further includes a spring screw fixed to the lever, the spring screw including a ball bearing, the ball bearing configured to move in and out of the indent with rotation of the deflection lever, and a shaft that is distal of the control handle and responsive to the deflection lever for bidirectional deflection.

In some embodiments, the bidirectional deflection of the shaft includes a greater deflection in one direction and a lesser deflection in an opposite direction.

In some embodiments, the greater deflection is about 180 degrees and the lesser direction is about 90 degrees.

In some embodiments, the deflection lever has one arm on one side of the longitudinal bisecting axis and another arm on an opposite side of the longitudinal bisecting axis.

In some embodiments, the deflection lever includes one arm with a tactilely uneven surface and another arm with a tactilely smooth surface.

In some embodiments, the deflection lever includes a boss, the spring screw extending through the boss.

In some embodiments, the boss is parallel with the rotational axis.

In some embodiments, the boss is perpendicular to the longitudinal bisecting axis.

In some embodiments, the boss is aligned with a bisecting axis of the deflection lever.

In some embodiments, the bisecting axis of the deflection lever aligns with the longitudinal bisecting axis of the control handle when the deflection lever is in the neutral position.

In some embodiments, the catheter includes a friction-inducing washer between the deflection lever and the outer surface.

In some embodiments, the catheter includes a visual indicator on the deflection lever in alignment with the spring screw.

### BRIEF DESCRIPTION OF THE DRAWINGS

These and other features and advantages of the present invention will be better understood by reference to the following detailed description when considered in conjunction with the accompanying drawings. It is understood that selected structures and features have not been shown in certain drawings so as to provide better viewing of the remaining structures and features.
FIG. 1 is a perspective view of a catheter with tactile deflection indication, according to an embodiment.
FIG. 2 is a perspective view of a control handle, according to an embodiment.
FIG. 3 is a top perspective view of a housing of the control handle, according to an embodiment.
FIGS. 4A, 4B and 4C are top plan views of the catheter of FIG. 1, with a deflection lever of a control handle in a neutral position, a position of lesser deflection and a position of greater deflection, respectively.
FIG. 5A is a top perspective view of a deflection lever, according to an embodiment.
FIG. 5B is a bottom perspective view of the deflection lever of FIG. 5A.
FIG. 5C is a bottom perspective of the deflection lever of FIG. 5A, shown with a friction-inducing washer.
FIG. 6 is a side perspective view of the housing of the control handle of FIG. 3, shown with a spring screw and a fiction-inducing washer.
FIG. 7 is a side cross-sectional view of a spring screw, engaged with an indent of the housing of the control handle, according to an embodiment.
FIG. 8A is a top perspective view of a housing of the control handle, according to an alternate embodiment.
FIG. 8B is a bottom perspective view of a deflection lever, according to an alternate embodiment.

### DETAILED DESCRIPTION OF THE PREFERRED EMBODIMENTS

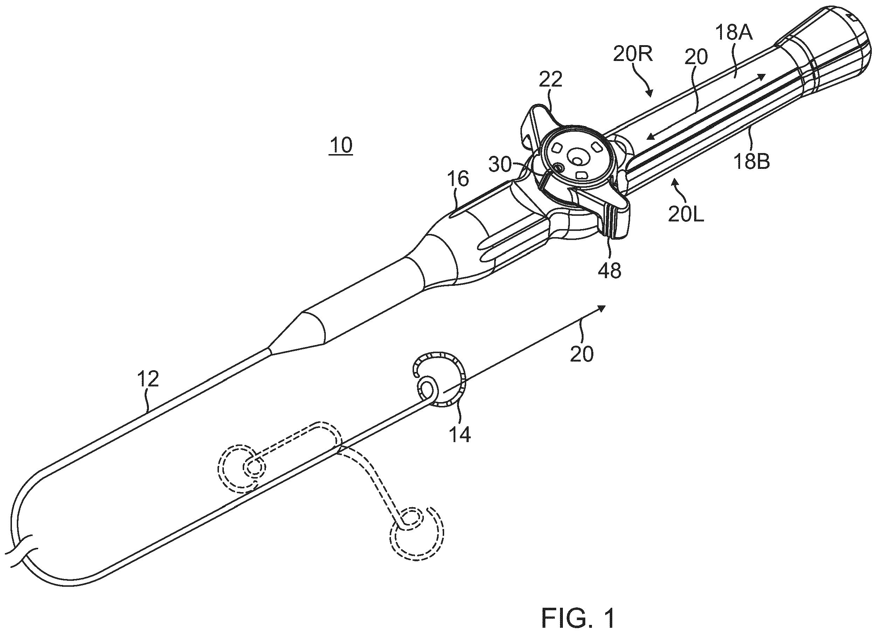

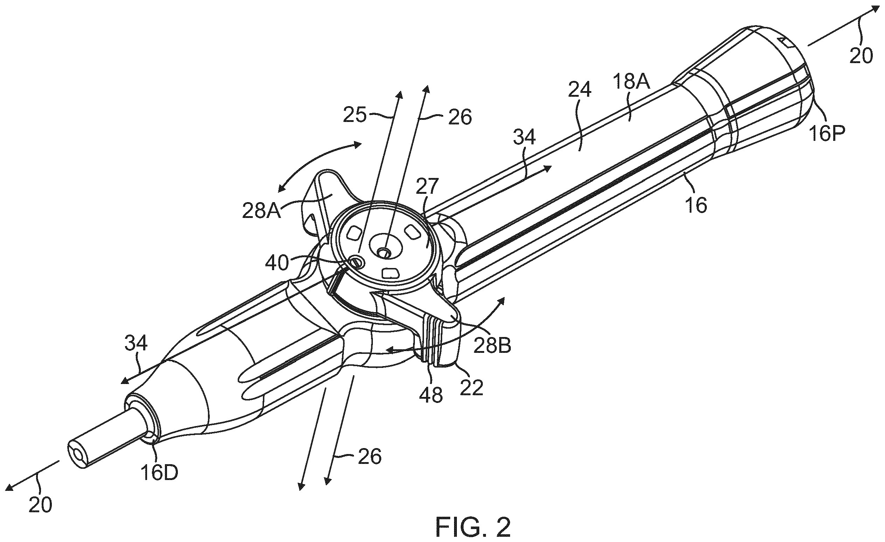

Referring to **FIG. 1** and **FIG. 2****,** in some embodiments, a deflection catheter 10 includes an elongated deflectable shaft 12, a distal effector 14, for example, a "lasso" mapping assembly with electrodes, and a control handle 16 proximal of shaft 12 for controlling deflection of the shaft 12.

As known in the art, the shaft 12 includes a multi-lumened tubing configured with at least two lumens, generally diametrically-opposed to each other, through each of which extends a respective puller wire that is actuated by mechanisms of the control handle, including a deflection lever 22. Such shaft and control handle mechanisms are described in U.S. Patent No. 9682214, titled "Control Handle With Rotational Cam Mechanism For Contraction/Deflection of Medical Device," the entire contents of which are incorporated herein by reference.

The control handle 16 includes a housing 18 that may be formed by a first or upper portion 18A and a second or lower portion 18B which together enclose an interior space within the control handle. The control handle 16 defines a center longitudinal axis 20 which bisects the control handle into generally symmetrical elongated halves, namely, left half 20L and right half 20R.

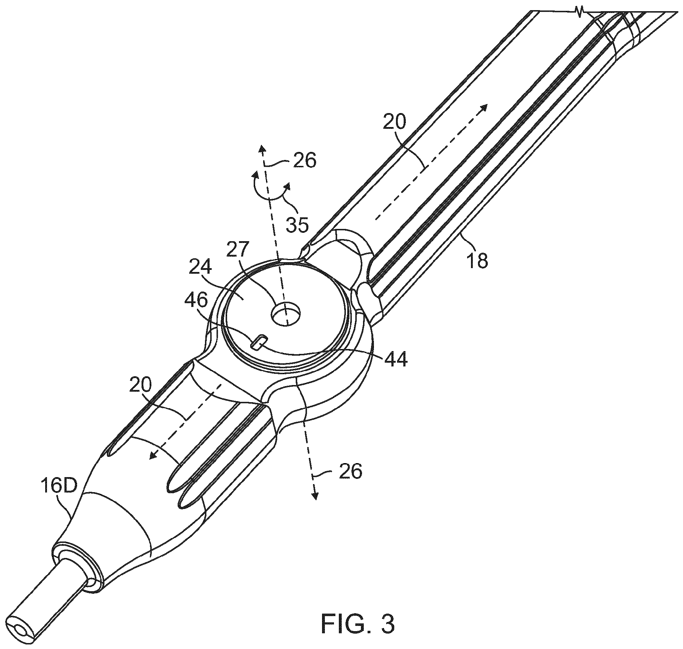

As shown in **FIG. 3****,** the upper portion 18A of the housing has a region of a generally flat outer surface 24 over which the deflection lever 22 is mounted for attachment to the control handle 16. In some embodiments, the outer surface 24 is located closer to distal end 16D of the control handle compared to proximal end 16P, for example, at greater than two-thirds the length of the control handle closer to the distal end 16D than the proximal end 16P. Because the deflection lever 22 is mounted over the outer surface 24, the location of the outer surface 24 reflects ergonomic considerations in the configuration of the control handle 16 as a device to be held in and manipulated by an operator's hand.

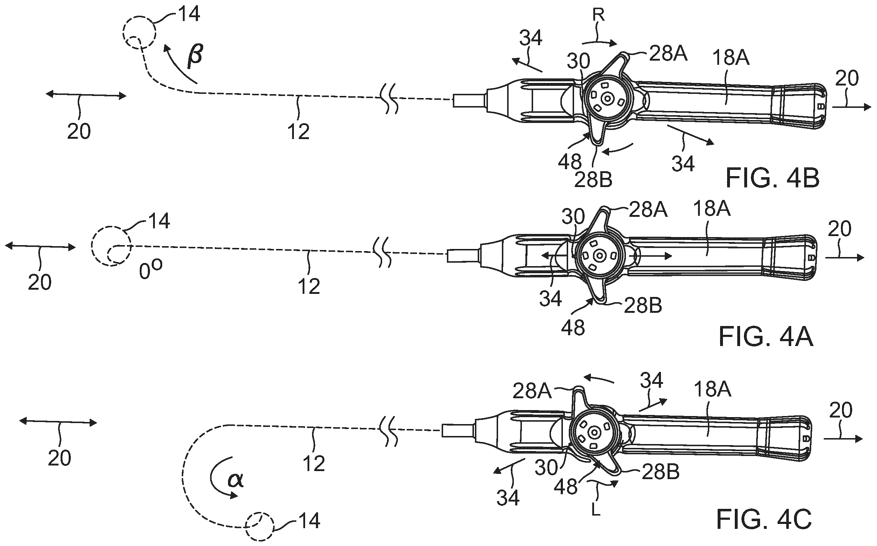

In the illustrated embodiment, the outer surface 24 is defined by a circular area. Formed in the upper portion 18A within the region of the outer surface 24 is an opening 27 that is centered relative to the outer surface 24 and configured to receive a bolt (not shown) that secures the deflection lever 22 to the control handle 16 while allowing rotation of the deflection lever 22 relative to the housing 18 about a rotational axis 26 defined by the opening 27. The rotational axis 26 is generally perpendicular to the longitudinal axis 20 and the deflection lever 22 can be rotated by a user in opposing directions (arrow 35) about the rotational axis 26 in deflecting the catheter shaft 12 correspondingly in opposing directions to the right and the left of the longitudinal axis 20 (**FIG. 1**). Again, U.S. Patent 9682214 which has been incorporated hereinabove by reference discloses deflection mechanisms within the control handle that act on puller wires in response to a user's manipulation of the deflection lever in deflecting the catheter shaft bidirectionally. As shown in **FIG. 4A****,** the catheter shaft 12 is undeflected when the deflection lever 22 is in a neutral position defined by alignment with the longitudinal axis 20. As shown in **FIG. 4B****,** the catheter shaft 12 is deflected to the right of the longitudinal axis 20 at angle β when the deflection lever 22 has been rotated to the right (see arrows R). As shown in **FIG. 4C****,** the catheter shaft 12 is deflected to the left of the longitudinal axis 20 at angle α when the deflection lever 22 has been rotated to the left (see arrows L).

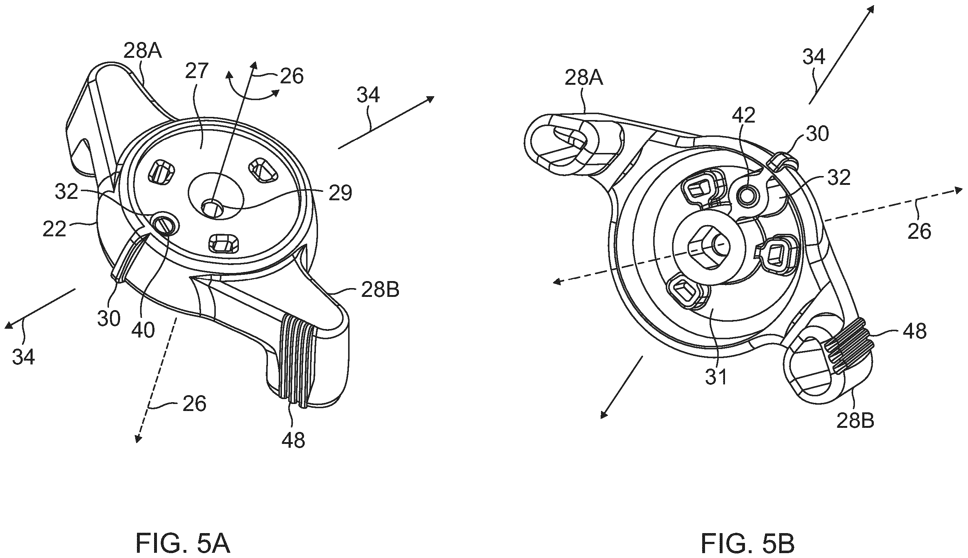

In some embodiments, the deflection lever 22 has a bisecting axis 34 that bisects the lever into two symmetrical halves (e.g., a right half and a left half), as shown in **FIG. 5A** and **FIG. 5B****.** The bisecting axis 34 is aligned with the longitudinal axis 20 of the control handle when the deflection lever 22 is in the neutral position (see **FIG. 2**). The deflection lever 22 has two arms 28A, 28B that are on opposite sides of the axis 34, with one arm on a respective side of the control handle 16. On a distal outer surface of the deflection lever 22, positioned on the axis 34, is a visual marker 30 that is centered between the arms 28A, 28B. The visual marker 30 is aligned with the longitudinal axis 20 of the control handle 16 when the deflection lever 22 is in the neutral position (see **FIG. 4A**) and points away to either side of the neutral position when the deflection lever has been rotated (see **FIG 4B** and **FIG. 4C**). The deflection lever 22 is configured to at least encourage operators to hold the control handle 16 in their hand with their thumb and index finger placed on a distal surface of a respective arm 28A, 28B, where the deflection lever 22 can be rotated by movement of the operators' thumb and/or index finger in opposite directions about the rotational axis 26.

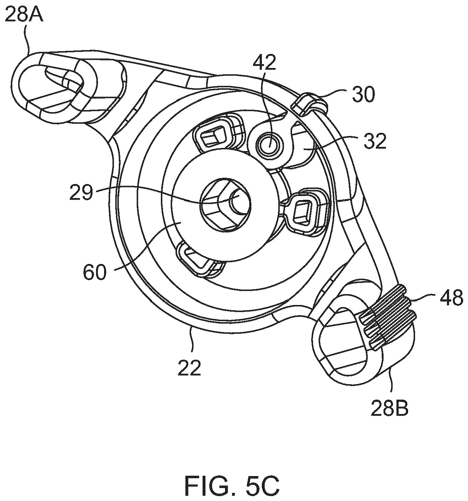

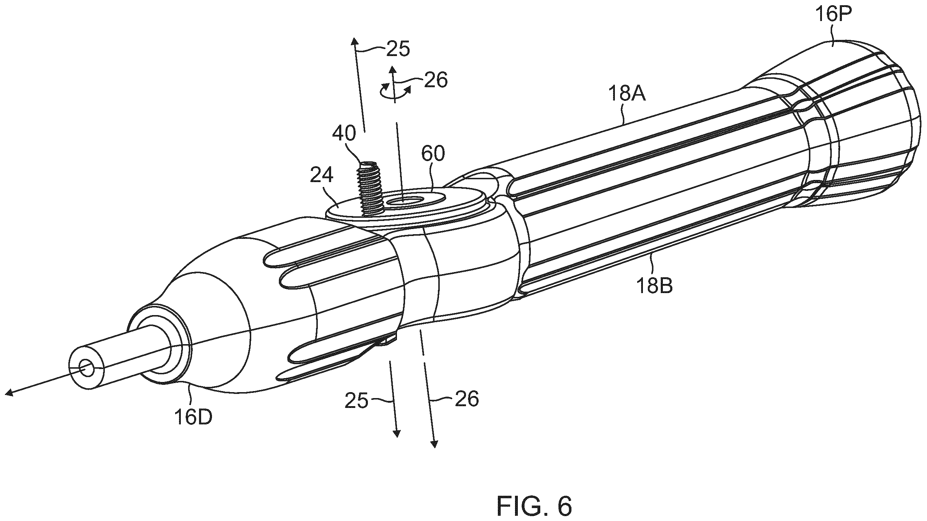

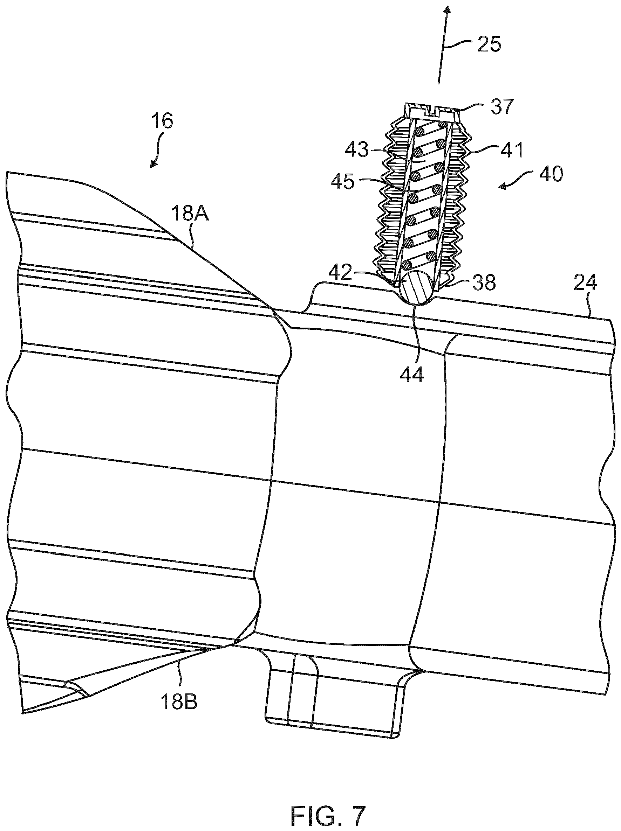

With reference to **FIG. 5B****,** **FIG. 5C** and **FIG. 6****,** a threaded boss 32 is formed in an underside of the deflection lever 22. The boss 32 defines a through-hole that extends from the underside through to outer surface 27 of deflection lever 22 and defines an axis 25 that is parallel to the rotational axis 26 of the deflection lever 22 and thus perpendicular to the outer surface 27 of deflection lever 22. Inserted in the boss 21 are a spring screw 40 (**FIG. 6**), and a ball bearing 42 (**FIG. 5C**) that is situated between the spring screw 40 and the outer surface 24 of the control handle housing 18A under the deflection lever 22. Biased by the elastic spring force of the spring screw 40 in a direction toward the outer surface 24, the ball bearing 42 maintains contact with the outer surface 24 in all rotational positions of the deflection lever 22 about the axis 26. As shown in **FIG. 7****,** in some embodiments, the spring screw 40 has a head 37, an end 38 opposite of the head, and a threaded exterior 41 defining an interior core 43 along its length, that contains an elastic force member 45, e.g., a spring coil or a compressible fluid, that acts on the ball bearing 42 at the end 38 of the spring screw 40. The threated exterior 41 enables the spring screw 40 to be fixed within the boss 32, where its position (or depth) in the boss can be adjusted by turning of the spring screw 40 at the head 37 by a tool such as a screwdriver. While the spring screw 40 is adjustably fixed in the boss, the ball bearing 42 under the elastic force of the force member 45 in the core 43 is movable longitudinally along the axis 25 and has "spring" action relative to the threaded exterior 41.

As shown in **FIG. 3****,** the outer surface 24 of the control handle housing 18A is advantageously configured with an indent 44 that is aligned with a center longitudinal axis 34 (**FIG. 2**) of the control handle 20, and also with the longitudinal axis 34 of the deflection lever 22 when the deflection lever 22 is in the neutral position. In particular, when the deflection lever 22 is in the neutral position, the spring screw 40 and ball bearing 42 are in perpendicular alignment with the indent 44 such that the ball bearing 42 enters the indent 44 under the elastic force of the spring screw 40. The indent 44 is sized and shaped to receive the ball bearing 42, yet also to allow the ball bearing 42 to exit the indent when the operator rotates the deflection lever 22 about the axis 26 out of the neutral position (when deflecting the catheter shaft). Between the ball bearing 42 moving in and out of the indent 44 in response to the rotation of the deflection lever 22, the ball bearing 42 assumes an outer/protruded position relative to the threaded exterior 41 when the ball bearing 42 rests in the indent 44 with the deflection lever 22 in the neutral position (**FIG. 7**) and an inner/withdrawn position relative to the threaded exterior 41 when the ball bearing 42 rests against the outer surface 24 when the deflection lever 22 is in a rotated position.

Advantageously, the movement of the ball bearing 42 into and out of the indent 44 relative to the surrounding flat outer surface 24 provides a tactile indication to the operator's thumb and index finger when the deflection lever 22 is in or out of the neutral position and correspondingly when the catheter shaft 12 is in or out of deflection. In some embodiments, the tactile indication or "feel" presented to the operator may include, for example, a "click" or other slight detectable change in the ease or resistance of rotation of the deflection lever 22 when the ball bearing 42 slides in and out of the indent 44. It is understood that the elastic force member 45, the ball bearing 42 and/or the indent 44 can be configured to increase or decrease the tactile indication, for example, such that the operator is required to exercise a threshold amount of force with his thumb and index finger to rotate the deflection lever 22 in moving the ball bearing 42 into and/or out of engagement with the indent 44. In some embodiments, a friction-inducing washer 60 is positioned between the deflection lever 22 and the outer surface 24 (see **FIG. 5C** and **FIG. 6**) so that the deflection lever 22 can better maintain its rotational position wherever the operator may set it without the catheter shaft 12 losing its deflection and straightening out.

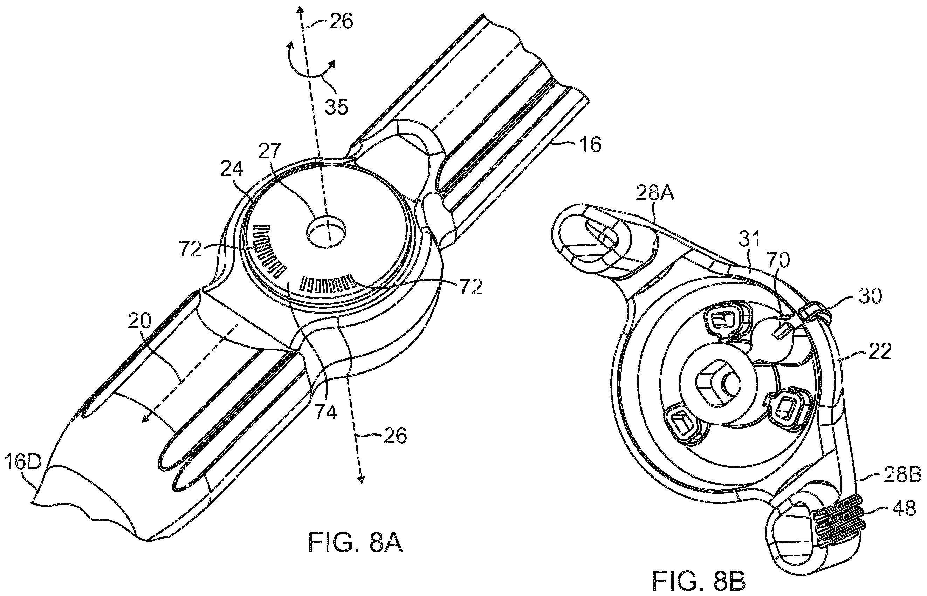

In alternate embodiments, as shown in **FIG. 8A** and **FIG. 8B****,** the underside of the deflection lever 22 is formed with a tab 70 that brushes against a texturized surface, for example, a plurality of protrusions or fins 72 formed on the outer surface 24, on opposite sides of the axis 20, leaving a smooth gap 74 in an immediate region of the longitudinal axis 20. As such, the tab 70 contacts the ridges 72 only when the deflection lever 22 is rotated out of the neutral position, on either side of the center longitudinal axis 20. The engagement between the tab 70 and the ridges can provide both tactile and audio indications of the rotation and/or rotational position of the deflection lever 22.

In some embodiments, the deflection lever 22 provides a tactile indicator that is indicative of a direction of deflection, as shown in **FIG. 5A** and **FIG. 5B****.** On the outer surface of each arm 28A, 28B that contacts, respectively, the operator's thumb and index finger the outer surface of one arm is advantageously tactilely different from the other arm. In the illustrated embodiment, the outer surface of arm 28A is smooth whereas the outer surface of arm 28B is formed with ridges 48. With a right-handed user whose right palm is placed over the deflection lever 22 of a catheter with a greater deflection on one side (e.g., left angle α = 180 degrees) (**FIG. 4C**) and a lesser deflection on the other side (e.g., angle β = 90 degrees) (**FIG. 4B**), the thumb rests on the ridges 48 of the arm 28B and the index finger rests on the smooth surface of the arm 28A. Where the arm 28B is on the side of the greater deflection (e.g., angle α = 180 degrees), the ridges 48 tactilely inform the operator that his thumb controls the greater deflection side of the catheter and that his index finger controls the lesser deflection side of the catheter (e.g., angle β = 90). It is understood that in whatever manner the operator may grip the control handle 16 or manipulate the deflection lever 22, whether the operator is right-handed or left-handed, or whether the operator is using the control handle right-side-up (with the deflection lever 22 visible) or upside-down (with deflection lever 22 not visible), the tactile indicator, e.g., the ridges 48, tactilely distinguishes one arm from the other arm.

The control handle housing 16 and the deflection lever 22 may be manufactured by injection molding. It is understood that the boss 32 can be configured anywhere in the underside 31 of the deflection lever where it does not interfere with other components of the deflection lever or the control handle. In the illustrated embodiment, the boss 32 is configured in alignment with the visual marker 30 and lies on the longitudinal bisecting axis 34 so that the deflection lever remains symmetrical about the axis 34 for simplifying the injection molding process by which the deflection lever can be manufactured.

The preceding description has been presented with reference to presently preferred embodiments of the invention. Workers skilled in the art and technology to which this invention pertains will appreciate that alterations and changes in the described structure may be practiced without meaningfully departing from the principal, spirit and scope of this invention. Any feature or structure disclosed in one embodiment may be incorporated in lieu of or in addition to other features of any other embodiments, as needed or appropriate. As understood by one of ordinary skill in the art, the drawings are not necessarily to scale. Accordingly, the foregoing description should not be read as pertaining only to the precise structures described and illustrated in the accompanying drawings, but rather should be read consistent with and as support to the following claims which are to have their fullest and fair scope.

## Claims

1. A catheter comprising:
a control handle including an outer surface with an indent;
a deflection lever mounted on the control handle over the indent, the deflection lever configured for rotation relative to the control handle;
a ball bearing;
a spring screw configured to move the ball bearing in and out of engagement with the indent with rotation of the deflection lever; and
a shaft distal of the control handle, the shaft responsive to the deflection lever for bidirectional deflection.

2. The catheter of claim 1, wherein the ball bearing is between the spring screw and the outer surface and the spring screw is configured to urge the ball bearing toward the outer surface.

3. The catheter of claim 1, wherein the control handle defines a longitudinal axis and the deflection lever is configured for rotation about an axis perpendicular to the longitudinal axis.

4. The catheter of claim 1, wherein the deflection lever is configured with a boss through which the spring screw extends.

5. The catheter of claim 1, wherein the deflection lever has a first arm with a tactilely smooth surface and a second arm with a tactilely ridged surface, optionally wherein the shaft is undeflected when the deflection lever is in a neutral position.

6. The catheter of claim 1, wherein the deflection lever is in a neutral position when the ball bearing is engaged with the indent, optionally wherein the shaft is deflected when the deflection lever is in a rotated position..

7. The catheter of claim 1, wherein the deflection lever is in a rotated position when the ball bearing is out engagement with the indent.

8. A catheter comprising:
a control handle defining a longitudinal bisecting axis and including an outer surface with an indent aligned with the longitudinal bisecting axis;
a deflection lever mounted on the control handle, the deflection lever configured for bidirectional rotation relative to the longitudinal bisecting axis about a rotational axis perpendicular to the longitudinal bisecting axis, the indent defining a neutral position of the bidirectional rotation of the deflection lever;
a spring screw fixed to the lever, the spring screw including a ball bearing, the ball bearing configured to move in and out of the indent with rotation of the deflection lever; and
a shaft distal of the control handle, the shaft responsive to the deflection lever for bidirectional deflection.

9. The catheter of claim 8, wherein the bidirectional deflection of the shaft includes a greater deflection in one direction and a lesser deflection in an opposite direction, optionally wherein the greater deflection is about 180 degrees and the lesser direction is about 90 degrees.

10. The catheter of claim 8, wherein the deflection lever has one arm on one side of the longitudinal bisecting axis and another arm on an opposite side of the longitudinal bisecting axis, or includes one arm with a tactilely uneven surface and another arm with a tactilely smooth surface.

11. The catheter of claim 8, wherein the deflection lever includes a boss, the spring screw extending through the boss.

12. The catheter of claim 11, wherein the boss is i) parallel with the rotational axis, or ii) perpendicular to the longitudinal bisecting axis, optionally wherein the boss is aligned with a bisecting axis of the deflection lever, further optionally wherein the bisecting axis of the deflection lever aligns with the longitudinal bisecting axis of the control handle when the deflection lever is in the neutral position.

13. The catheter of claim 8, further comprising a friction-inducing washer between the deflection lever and the outer surface, or a visual indicator on the deflection lever in alignment with the spring screw.

14. A catheter, comprising:
a shaft;
a control handle with a housing configured with an indent; and
a deflection lever mounted on the housing over the indent and configured to deflect the shaft with rotation of the deflection lever, the deflection lever including a ball bearing and a spring screw configured to mobilize the ball bearing into elastic contact with the indent when the deflection lever is rotated into a neutral position such that the elastic contact provides a tactile indication of shaft deflection detected through the deflection lever.

15. The catheter of claim 14, wherein the ball bearing has elastic contact with the housing when the deflection lever is rotated out of the neutral position, or the deflection lever includes a boss through which the spring screw extends.

16. The catheter of claim 14, wherein the indent is aligned with a longitudinal axis of the control handle, optionally wherein the spring screw includes a head and an end that together define an axis that is generally perpendicular to the longitudinal axis, and the ball bearing is between the end and the indent.
